# EUROPÄISCHE PATENTSCHRIFT

(11) **EP 2 428 243 B1**
(45) Veröffentlichungstag und Bekanntmachung des Hinweises auf die Patenterteilung: **12.10.2016**
(21) Anmeldenummer: 11007005.9
(22) Anmeldetag: 27.08.2011
(51) Int. Cl.: A61M 16/06, A61M 16/08

(54) **Leckagenventil**
Leak valve
Soupape anti-fuites

(30) Priorität: 14.09.2010 DE 102010045299
(43) Veröffentlichungstag der Anmeldung: 14.03.2012
(73) Patentinhaber: WILAmed GmbH, 91126 Kammerstein (DE)
(72) Erfinder: Lanfranchi, Marcello, 91126 Schwabach (DE)
(74) Vertreter: Stippl, Hubert

(56) Entgegenhaltungen:
- EP-A1- 1 362 610
- EP-A2- 1 314 446
- DE-A1-102005 041 716

## Beschreibung

Die vorliegende Erfindung betrifft ein Leckagenventil gemäß dem Oberbegriff des Anspruchs 1.

### Technologischer Hintergrund

Leckagenventile werden bei der sogenannten Leckagenbeatmung eingesetzt. Hierbei bekommt der zu beatmende Patient von einem Beatmungsgerät eine Menge an Beatmungsgas zur Verfügung gestellt, die größer ist als die vom Patienten bei der Atmung verbrauchten Menge. Der Rest dieser vom Beatmungsgerät zur Verfügung gestellten Beatmungsgasmenge verlässt den Beatmungskreis über das Leckagenventil. Bei der Inspiration durchströmt das Beatmungsgas das Innenlumen des Leckagenventils und wäscht zugleich auch alle Reste an verbrachter Luft aus dem Leckagenventil heraus. Das Beatmungsgerät besitzt eine exakt definierte Förderleistung an Beatmungsgas. Diese Förderleistung kann an die betreffenden patientenabhängigen Verbrauchsmengen (z.B. Beatmung eines Erwachsenen oder eines Säuglings) geräteseitig angepasst werden. Beatmungsgeräte zur Leckagenbeatmung sind üblicherweise genau auf die durch das Leckagenventil festgelegte Leckagenmenge abgestimmt. Sofern es zu Schwankungen der Leckagenmenge kommt, werden diese nicht erfasst und führen daher zu einer nicht gewünschten Beeinträchtigung der Beatmung, was insbesondere bei der Beatmung von Neugeborenen von besonderer Wichtigkeit ist.

### Nächstliegender Stand der Technik

Aus der EP 1 362 610 A1 ist ein Leckagenventil gemäß dem Oberbegriffs des Anspruchs 1 bekannt. Die Besonderheit dieses Leckagenventils besteht darin, dass eine Mehrzahl von Distanzelementen zwischen einem Sockelsegment und einem Anschlusssegment vorgesehen sind, wobei mindestens eines der Distanzelemente zur Erzielung einer gleichmäßigen Strömungsführung als Radialsteg ausgebildet ist, der sich bezüglich einer Strömungslängsachse in radialer Richtung hierzu erstreckt. Die Verrastung des Innenteils mit dem Außenteil erfolgt über mindestens zwei Federlaschen, die im stirnseitigen Ende des Außenteils angreifen. Innenteil und Außenteil sind gegeneinander verdrehbar. Üblicherweise ist jedoch bei einer derartigen Rastverbindung ein gewisses axiales sowie radiales Spiel von Innenteil zu Außenteil vorhanden, welches während des Betriebs durch die mechanische Beeinträchtigung bei der Handhabung zu erheblichen Schwankungen der Leckagenmenge führt.

Aus der US 5 937 851 ist ein Leckagenventil bekannt, welches ebenfalls aus einem Außenteil sowie einem damit verrastbaren Innenteil besteht. Das Innenteil verfügt auch hier über Federlaschen, die am stirnseitigen Ende des Außenteils angreifen. Innenteil und Außenteil sollen auch hierbei bewusst gegeneinander verdrehbar sein. Auch hier besteht das Problem, dass bei mechanischer Beeinträchtigung des Leckagenventils es zu erheblichen Schwankungen der Leckagenmenge kommt.

### Aufgabe der vorliegenden Erfindung

Die Aufgabe der vorliegenden Erfindung besteht darin, ein neuartiges Leckagenventil zur Verfügung zu stellen, welches einfach im Aufbau ist und eine reduzierte Schwankungsbreite der Leckagenmenge gewährleistet.

### Lösung gemäß der Erfindung

Die vorstehende Aufgabe wird bei dem gattungsgemäßen Leckagenventil dadurch gelöst, dass im verrasteten Zustand der beiden Gehäuseteile zusätzlich zur Rastverbindung eine Klemmverbindung vorgesehen ist und entlang des Umfangs der Ringfläche eine Mehrzahl von Anlagevorsprüngen vorgesehen ist. Insbesondere können die Rastlaschen und/oder die gekrümmten Wandbereiche in Bezug auf die Abmessungen des Anschlussstutzens derart dimensioniert sein, dass die Rastlaschen bzw. Wandbereiche im verrasteten Zustand des Leckagenventils mit der Wand des Anschlussstutzens verklemmen. Hierdurch wird ein Leckagenventil zur Verfügung gestellt, welches einerseits einfach im Aufbau sowie kostengünstig in der Herstellung ist als auch eine wirksame Reduzierung der Schwankungsbreite der Leckagenmenge gewährleistet, vor allem dann, wenn bei dem Einsatz des Leckagenventils durch an diesem beispielsweise durch Kopfbewegungen der zu beatmenden Person oder des Neugeborenen es zu einer mechanischen Beanspruchung der Verbindung der beiden Gehäuseteile kommt. Die Rastverbindung wird hierdurch gleichzeitig durch die Klemmverbindung unterstützt. Das Vorhandensein der Mehrzahl von die Kraft punktförmig einleitenden Anlagevorsprüngen entlang des Umfangs der Ringfläche führt ebenfalls zu einer Erhöhung der Klemmwirkung im Bereich der beiden Gehäuseteilstufungen und damit zu einer Reduzierung der Schwankungsbreite der Leckageströmung bei Einwirkung mechanischer Kräfte auf die beiden Gehäuseteile.

Die Idee gewährleistet es, dass die beiden Gehäuseteile im verrasteten Zustand ohne das Vorhandensein eines axialen Bewegungsspiels aneinander liegen.

Die vorliegende Erfindung ermöglicht es, bei einfachem Aufbau die Leckageströmung des Leckagenventils auf eine maximale Schwankungsbreite ±10 %, vorzugsweise ±8 %, besonders vorzugsweise auf ±6 % bezogen auf eine Soll-Leckagenmenge zu begrenzen.

Zweckmäßigerweise weisen die Anlagevorsprünge eine Höhe im Bereich von 0,1 mm bis 0,3 mm auf.

Eine noppenartige Form der Anlagevorsprünge hat sich hierbei besonders bewährt.

Die beiden Gehäuseteile sind durch die Verrast- und unterstützende Klemmverbindung derart miteinander verbunden, dass sie trotz ihrer Rotationssymmetrie im verrasteten Zustand nicht gegeneinander um die Längsachse des Gehäuses drehbar sind, d. h. die Rastverbindung und/oder Klemmverbindung einer freien Drehbarkeit der beiden Gehäuseteile zueinander entgegenwirkt.

Die im Bereich des Leckagekanals vorgesehene, einem Austrittsspalt vorgelagerte Staukammer für die Leckageströmung dient dazu, ein gewisses Puffervolumen innerhalb des Leckageventils vorzusehen, wodurch Schwankungen der Leckageströmung zusätzlich verringert werden können.

Dadurch, dass der Bereich des inneren Gehäuseteils, der Durchgangsöffnungen aufweist, parallel zur Längsachse des Ventilgehäuses verläuft, wird die Herstellbarkeit des Leckageventils vereinfacht.

### Ausführungsbeispiel der Erfindung

Nachstehend wird anhand von Zeichnungsfiguren ein zweckmäßiges Ausführungsbeispiel der Erfindung näher beschrieben. Es zeigen:
- Fig. 1: eine stark vereinfachte, schematische Darstellung der Positionierung des erfindungsgemäßen Leckagenventils im Einsatz;
- Fig. 2: eine Teilschnittdarstellung durch eine Ausgestaltung des erfindungsgemäßen Leckagenventils im zusammengesetzten Zustand;
- Fig. 3: eine Teilschnittdarstellung des ersten und zweiten Gehäuseteils des Leckagenteils nach Fig. 2 in getrenntem Zustand sowie
- Fig. 4: eine vergrößerte Teilschnittdarstellung des Bereichs des Leckagenkanals des Leckagenteils nach Fig. 2.

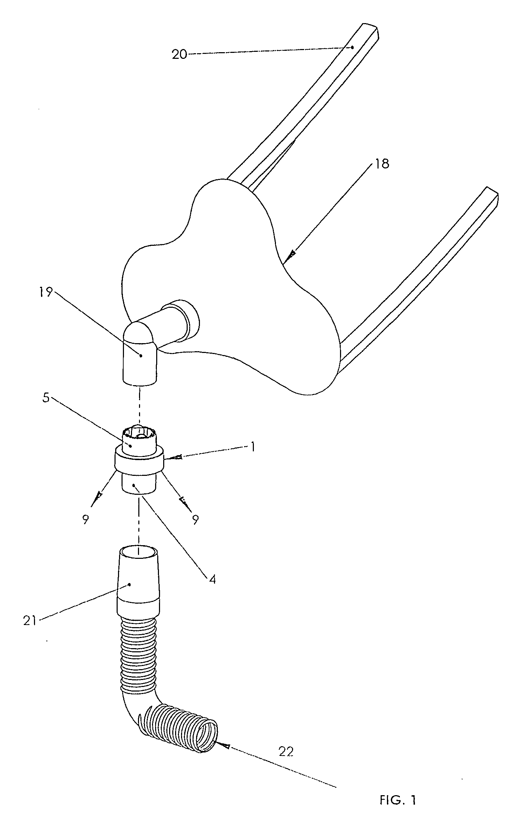

Bezugsziffer 1 in Fig. 1 bezeichnet das erfindungsgemäße Leckagenventil. Es ist patientenseitig positioniert, beispielsweise, wie in Fig. 1 dargestellt, an dem Anschlussstück 19 einer Beatmungsmaske 18 die mittels Haltebänder 20 am Kopf des Patienten bzw. Neugeborenen befestigt wird. Zum Anschluss des Leckagenventils 4 weist dieses einen ersten Anschlussstutzen 14 auf, der in das Anschlussstück 19 der Beatmungsmaske 18 eingesteckt wird.

An der gegenüberliegenden Seite befindet sich ein zweiter Anschlussstutzen 4 zur Verbindung mit einem Atemgasschlauch 21, der mit einem (nicht dargestellten) Beatmungsgerät (Leckagengerät) zur Erzeugung eines Atemgasstroms 22 verbunden ist.

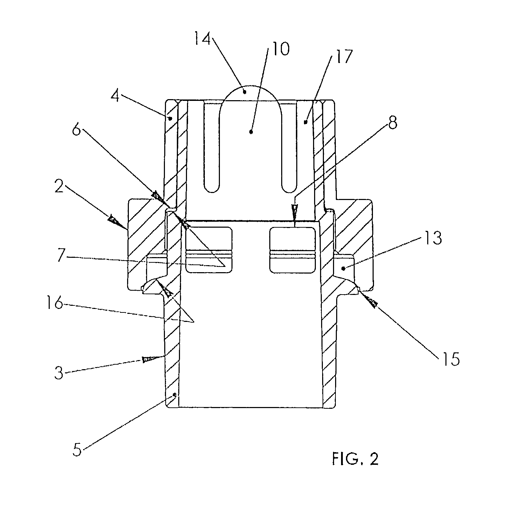

Im Leckagenventil 1 wird ein Teil des Atemgasstroms 22 als Leckagenstrom 9 nach außen abgeführt. Bei der Ausgestaltung des Leckagenventils 1 nach Fig. 1 erfolgt dies über einen ringförmigen Austrittspalt 15 (vgl. Fig. 2). Anstelle der Beatmungsmaske 18 kann auch eine Nasenbrille oder ein Tubus, letzterer für eine sogenannte invasive Beatmung, vorgesehen sein. In letzterem Fall wird das Leckagenventil mit einer sog. "Gänsegurgel" verbunden.

Das Leckagenventil ist, wie dies in Fig. 2 dargestellt ist, zweiteilig aufgeteilt. Es umfasst ein erstes Gehäuseteil 2 sowie zweites Gehäuseteil 3, welche ineinander gesteckt und axial fixiert werden. Die beiden Gehäuseteile 2, 3 werden ineinander gesteckt, indem entsprechend dimensionierte gekrümmte Wandabschnitte 17 sowie ebenfalls gekrümmt ausgebildete Rastlaschen 10 an der Innenwandung des ersten Anschlussstutzens 4 anliegend diesen durchsetzen, wobei eine am offenen Ende der Rastlasche 10 vorgesehenen Nase 14 das stirnseitige Ende des ersten Anschlussstutzens 4 umgreift und eine axiale Verrastung herbeiführt. Die beiden Gehäuseteile 2, 3 liegen insbesondere über eine erste Ringfläche 6 des ersten Gehäuseteils 2 sowie zweite Ringfläche 7 des zweiten Gehäuseteils 3 in Axialrichtung betrachtet aneinander an.

Etwa im Mittelbereich des Leckagenventils 1 befindet sich ein Erweiterungsbereich, gebildet durch einen radialen Vorsprung des ersten Gehäuseteils 2. Etwa in der Höhe dieses Erweiterungsbereichs sind beim zweiten Gehäuseteil 3 Durchgangsöffnungen 8 vorgesehen, die dazu dienen, einen Austritt von Atemgas aus dem Atemgasstrom in den Leckagekanal zu gewährleisten.

Der Leckagekanal wird durch einen zwischen erstem Gehäuseteil 2 und zweitem Gehäuseteil 3 gebildeten Ringspalt gebildet, der in eine ringförmig umlaufende Staukammer 13 mündet. Unterhalb der Staukammer 13 tritt ein umlaufender Vorsprung 16 hervor und bildet mit dem angrenzenden ersten Gehäuseteil 2 einen ringförmig umlaufenden Austrittsspalt 15 für eine Leckageströmung. Der umlaufende Vorsprung 16 ist an seiner Oberseite entsprechend abgerundet. Entsprechend abgerundet ist auch der unmittelbar gegenüberliegende Endbereich des ersten Gehäuseteils 2.

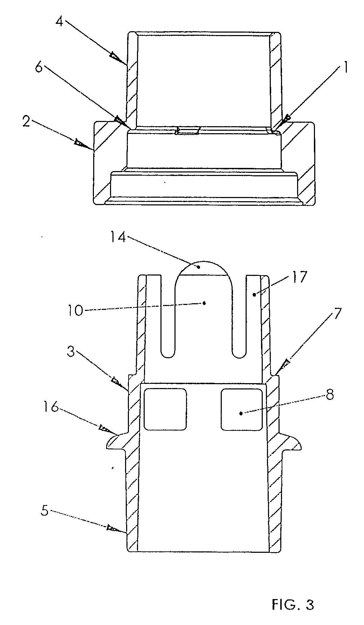

Wie aus Fig. 3 ersichtlich werden die beiden Gehäuseteile 2, 3 mittels der Rastlaschen 10 sowie mittels der gekrümmten Wandbereiche 17 miteinander verbunden.

Die Ausbildung und/oder Dimensionierung der Rastlaschen 10, der gekrümmten Wandbereiche 17 sowie der Innenwandung des ersten Gehäuseteils 2 ist erfindungsgemäß derart vorzunehmen, dass neben der Haltewirkung der Rastlaschen 10 eine zusätzliche Klemmwirkung sich einstellt, die dazu führt, dass die Schwankungsbreite des Leckagenstroms auch bei intensiver mechanischer Einflussnahme im wesentlichen konstant bleibt. Die beiden Gehäuseteile 2, 3 liegen im verrasteten Zustand ohne axialem Bewegungsspiel aneinander.

Die Erfindung ermöglicht es, die Leckageströmung des Leckageventils beispielsweise auf eine maximale Schwankungsbreite von ±4 l/min, vorzugsweise ±2 l/min, bezogen auf eine Soll-Leckagenstrommenge von z. B. 28-29 l/min zu senken.

Im Bereich der Ringflächen 6, 7 befinden sich eine Mehrzahl von Anlagevorsprünge 11, die eine besonders vorteilhafte Anlage der beiden Gehäuseteile 2, 3 zueinander und zwar auch bei mechanischer Einflussnahme gewährleistet. Diese können vorzugsweise noppenartige Form aufweisen.

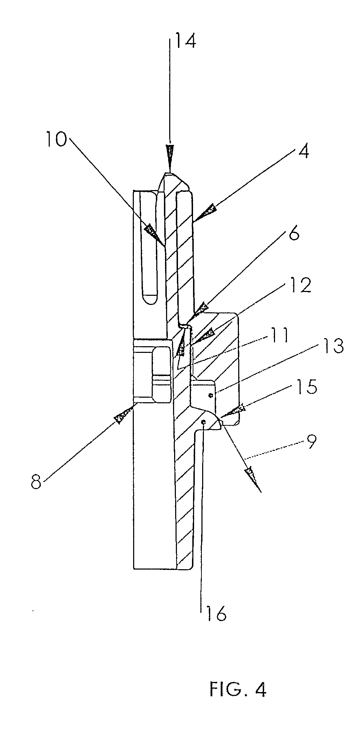

Wie aus Fig. 4 ersichtlich bedingen die Anlagevorsprünge 11, die vorzugsweise eine Höhe im Bereich von 0,1 mm bis 0,3 mm aufweisen eine entsprechende Beabstandung der ersten Ringfläche 6 zur zweiten Ringfläche 7. Darüber hinaus wird durch entsprechende Dimensionierung des ersten Gehäuseteils 2 zum zweiten Gehäuseteil 3 ein ringförmiger Kanalabschnitt 12 zwischen erstem Gehäuseteil 2 und zweitem Gehäuseteil 3 geschaffen, welcher in eine Staukammer 13 übergeht. Am Ende der Staukammer 13 befindet sicht ein ringförmiger Austrittsspalt 15, der durch einen umlaufenden, an der Oberseite gekrümmten Vorsprung 16 sowie durch eine unterseitige, ebenfalls entsprechend gekrümmte Stirnfläche des ersten Gehäuseteils 2 gebildet wird.

Aufgrund der Durchgangsöffnungen 8 strömt somit ein Teil des Atemgasstroms 22 aus dem Inneren des Leckagenventils 1 durch die Durchgangsöffnungen 8 hindurch in den Bereich des Kanalabschnitts 12 sowie der Staukammer 13 und von dort durch den ringförmigen Austrittspalt 15 hinaus ins Freie. Die Staukammer 13 wirkt in Verbindung mit dem ringförmigen Austrittspalt 15 eine gewisse Pufferwirkung und damit Vergleichmäßigung des Leckagestroms 9 aus.

Zur axialen Fixierung umgreifen die Rastlaschen 10 mit ihren endseitigen Nasen 14 des zweiten Gehäuseteils 3 das stirnseitige, oberseitige Ende des ersten Gehäuseteils 2. Hierdurch wird eine axiale Verrastung des ersten Gehäuseteils 2 sowie zweiten Gehäuseteils 3 erzielt. Ferner sind die Rastlaschen 10 und/oder die gekrümmten Wandbereiche 17 des zweiten Gehäuseteils 3 derart dimensioniert, dass die Rastlaschen 10 bzw. Wandbereiche 17 im verrasteten Zustand des Leckagenventils 1 mit der Wand des Anschlussstutzens 4 verklemmen, also eine zur Verrastung zusätzliche Klemmverbindung erzeugen. Das erste Gehäuseteil 2 und zweite Gehäuseteil 3 sind hierdurch im verrasteten Zustand nahezu ohne axiales und/oder radiales, vorzugsweise ohne jegliches axiales und radiales Spiel miteinander verbunden. Im Bereich der ersten Ringfläche 6 sowie zweiten Ringfläche 7 tragen die entlang des Umfangs angeordneten Anlagevorsprünge 11 dazu bei, diese Lagefixierung zu unterstützen, wobei gleichwohl zwischen dem ersten Gehäuseteil 2 sowie zweiten Gehäuseteil 3 ein umlaufender Kanalabschnitt 12 für die Leckagenströmung vorgesehen ist.

Die Leckageströmung des erfindungsgemäßen Leckageventils weist lediglich eine maximale Schwankungsbreite von 10 l / min, vorzugsweise 5 l / min auf.

Die Verrastung und zusätzliche Verklemmung führt dazu, dass das erste Gehäuseteil 2 und zweite Gehäuseteil 3 im zusammengesetzten, d. h. verrasteten Zustand aufgrund der Klemmwirkung nicht, zumindest nicht mit normalem Kraftaufwand, drehbar sind d. h. die Rastverbindung und/oder Klemmverbindung einer freien Drehbarkeit der beiden Gehäuseteile zueinander entgegenwirkt..

Die Erfindung gewährleistet bei im Einwirken von mechanischen Lastungen auf das Leckagenventil 1, beispielsweise bedingt durch Kopfbewegungen der zu beatmenden Person bzw. des zu beatmenden Säuglings bzw. Neugeborenen sehr viel niedrigere Leckagestromschwankungen als bei herkömmlichen Leckageventilen mit vergleichbarem einfachem Aufbau.

Das Leckagenventil besteht aus Kunststoff, vorzugsweise aus NAF 21 (DuPont).

### BEZUGSZEICHENLISTE

- 1: Leckagenventil
- 2: erstes Gehäuseteil
- 3: zweites Gehäuseteil
- 4: erster Anschlussstutzen
- 5: zweiter Anschlussstutzen
- 6: erste Ringfläche
- 7: zweite Ringfläche
- 8: Durchgangsöffnung
- 9: Leckagestrom
- 10: Rastlasche
- 11: Anlagevorsprung
- 12: Kanalabschnitt
- 13: Staukammer
- 14: Nase
- 15: Austrittsspalt
- 16: umlaufender Vorsprung
- 17: gekrümmter Wandbereich
- 18: Beatmungsmaske
- 19: Anschlussstück
- 20: Halteband
- 21: Atemgasschlauch
- 22: Atemgasstrom

## Patentansprüche

1. Leckagenventil für den Einsatz in einem Patientenbeatmungssystem mit
einem aus einem ersten (2) und zweiten Gehäuseteil (3) aufgebautem, vorzugsweise aus Kunststoff bestehenden Ventilgehäuse, wobei beide Gehäuseteile einen vorzugsweise im Querschnitt kreisförmigen Fluidkanal bilden und das Ventilgehäuse umfasst:
einen ersten Anschlussstutzen (4) am ersten Gehäuseteil (2),
einen zweiten Anschlussstutzen (5) am zweiten Gehäuseteil (3) sowie
einen gemeinsamen, radial nach außen vorspringenden Gehäusebereich zwischen den beiden Anschlussstutzen (4, 5),
einer im ersten Gehäuseteil vorgesehenen inneren Ringfläche (6), die im verbundenen Zustand der beiden Gehäuseteile (2, 3) einer am zweiten Gehäuseteil vorgesehenen inneren Ringfläche (7) gegenüberliegt,
mindestens einer vorzugsweise einer Mehrzahl entlang des Umfangs angeordneter Durchgangsöffnungen (8), die etwa in der Höhe des nach außen vorspringenden Gehäusebereichs angeordnet sind und einen Austritt des Fluids aus dem Fluidkanal in Form einer Leckageströmung ermöglicht bzw. ermöglichen,
einem ebenfalls etwa in der Höhe des nach außen vorspringenden Gehäusebereichs angeordneten, zumindest entlang eines Teils vorzugsweise entlang des gesamten Umfangs des Gehäuses umlaufenden, durch beide Gehäuseteile (2, 3) gebildeten ringförmigen Leckagekanals (9) für die Leckageströmung,
einem Verrastmechanismus, mittels dem die beiden Gehäuseteile (2, 3) miteinander verbindbar sind,
als Rastmechanismus mindestens eine, vorzugsweise einer Mehrzahl von entsprechend dem Innenradius des Anschlussstutzens (4) gekrümmten Rastlaschen (10) mit stirnseitiger Nase (14) vorgesehen sind, die im Zustand der Verrastung sich entlang des Anschlussstutzens (4) innenseitig anliegend erstrecken und mit ihrer Nase (14) an der äußeren Stirnseite des Anschlussstutzen (4 bzw. 5) angreifen und eine axiale Verrastung gewährleisten und zwischen den Rastlaschen (10) entsprechend dem Innenradius des Anschlussstutzens (4) gekrümmte Wandbereiche (17) vorgesehen sind, die an der Innenfläche des Anschlussstutzens (4 bzw. 5) anliegen, wobei
im verrasteten Zustand der beiden Gehäuseteile (2, 3) zusätzlich zur Rastverbindung eine Klemmverbindung vorgesehen ist, **dadurch gekennzeichnet, dass**
entlang des Umfangs der Ringfläche (6 oder 7) eine Mehrzahl von Anlagevorsprüngen (11) vorgesehen ist.

2. Leckagenventil nach Anspruch 1,
**dadurch gekennzeichnet, dass**
die beiden Gehäuseteile (2, 3) im verrasteten Zustand ohne axiales Bewegungsspiel aneinanderliegen.

3. Leckagenventil nach Anspruch 1,
**dadurch gekennzeichnet, dass**
die Anlagevorsprünge (11) eine Höhe in einem Bereich von 0,1 mm - 0,3 mm aufweisen.

4. Leckagenventil nach Anspruch 1,
**dadurch gekennzeichnet, dass**
die Anlagevorsprünge (11) eine noppenartige Form besitzen.

5. Leckagenventil nach mindestens einem der vorhergehenden Ansprüche,
**dadurch gekennzeichnet, dass**
im Bereich des Leckagekanals (9) eine Staukammer (13) vorgesehen ist.

6. Leckagenventil nach mindestens einem der vorhergehenden Ansprüche,
**dadurch gekennzeichnet, dass**
der Bereich des inneren Gehäuseteils (3), das die Durchgangsöffnungen aufweist, parallel zur Längsachse des Ventilgehäuses verläuft.

## Claims

1. Leak valve for use in a patient ventilation system, with
a valve housing composed of a first housing part (2) and of a second housing part (3) and preferably made of plastic, wherein both housing parts form a fluid channel, which is preferably circular in cross section, and the valve housing comprises:
a first connector stub (4) on the first housing part (2),
a second connector stub (5) on the second housing part (3), and
a common, radially outwardly protruding housing area between the two connector stubs (4, 5),
an inner annular surface (6) provided in the first housing part, which annular surface (6), in the connected state of the two housing parts (2, 3), lies opposite an inner annular surface (7) provided on the second housing part,
at least one through-opening (8), preferably a plurality of through-openings (8), arranged along the circumference, which through-openings (8) are arranged approximately at the height of the outwardly protruding housing area and permit a discharge of the fluid from the fluid channel in the form of a leakage stream,
an annular leakage channel (9) for the leakage stream, which leakage channel (9) is likewise arranged approximately at the height of the outwardly protruding housing area, extends along at least part of the circumference of the housing, preferably along the entire circumference of the housing, and is formed by both housing parts (2, 3),
a locking mechanism, by means of which the two housing parts (2, 3) can be connected to each other,
at least one latching tab (10), preferably a plurality of latching tabs (10), curved according to the inner radius of the connector stub (4) and provided as latching mechanism, which latching tabs (10) have a lug (14) at the front and, in the locked state, extend along and bear on the inside of the connector stub (4) and engage with their lug (14) on the outer front face of the connector stub (4 or 5) and ensure axial locking, and wall areas (17), which are curved according to the inner radius of the connector stub (4), are provided between the latching tabs (10) and bear on the inner surface of the connector stub (4 or 5), wherein, in the locked state of the two housing parts (2, 3), a clamping connection is formed in addition to the locking connection, **characterized in that** a plurality of bearing projections (11) are provided along the circumference of the annular surface (6 or 7).

2. Leak valve according to Claim 1, **characterized in that** the two housing parts (2, 3), in the locked state, bear on each other without axial movement play.

3. Leak valve according to Claim 1, **characterized in that** the bearing projections (11) have a height in the range of 0.1 mm to 0.3 mm.

4. Leak valve according to Claim 1, **characterized in that** the bearing projections (11) have a knob-like shape.

5. Leak valve according to at least one of the preceding claims, **characterized in that** a storage chamber (13) is provided in the area of the leakage channel (9).

6. Leak valve according to at least one of the preceding claims, **characterized in that** the area of the inner housing part (3), which has the through-openings, extends parallel to the longitudinal axis of the valve housing.

## Revendications

1. Soupape antifuites à utiliser dans un système respiratoire d'un patient, avec
un corps de soupape composé d'une première partie de corps (2) et d'une deuxième partie de corps (3), constitué de préférence de matière plastique, dans lequel les deux parties de corps forment un canal de fluide ayant une section transversale de préférence circulaire, et le corps de soupape comprend:
un premier embout de raccordement (4) sur la première partie de corps (2),
un deuxième embout de raccordement (5) sur la deuxième partie de corps (3), ainsi que
une région de corps commune saillante radialement vers l'extérieur entre les deux embouts de raccordement (4, 5),
une face annulaire intérieure (6) prévue dans la première partie de corps, qui dans l'état assemblé des deux parties de corps (2, 3) est opposée à une face annulaire intérieure (7) prévue sur la deuxième partie de corps,
au moins une de préférence une multiplicité d'ouverture(s) de passage (8) disposée(s) le long de la périphérie, qui est/sont disposée(s) environ à la hauteur de la région de corps saillante vers l'extérieur et qui permet(tent) une sortie du fluide hors du canal de fluide sous la forme d'un écoulement de fuite,
un canal de fuite annulaire (9) pour l'écoulement de fuite, également disposé environ à la hauteur de la région de corps saillante vers l'extérieur, s'étendant au moins le long d'une partie, de préférence le long de toute la périphérie du corps et formé par les deux parties de corps (2, 3),
un mécanisme de verrouillage, au moyen duquel les deux parties de corps (2, 3) peuvent être assemblées l'une à l'autre,
il est prévu comme mécanisme de verrouillage au moins une, de préférence une multiplicité de patte(s) de verrouillage (10) incurvée(s) selon le rayon intérieur de l'embout de raccordement (4), avec un ergot frontal (14), qui dans l'état de verrouillage s'étendent le long de l'embout de raccordement (4) en s'appliquant sur le côté intérieur et qui s'accrochent avec leur ergot (14) sur le côté frontal extérieur de l'embout de raccordement (4 ou 5) et garantissent un verrouillage axial et il est prévu entre les pattes de verrouillage (10) des régions de paroi (17) incurvées selon le rayon intérieur de l'embout de raccordement (4), qui s'appliquent sur la face intérieure de l'embout de raccordement (4 ou 5), dans laquelle dans l'état verrouillé des deux parties de corps (2, 3), il est en outre prévu un dispositif de serrage en plus du dispositif de verrouillage, **caractérisée en ce qu'**il est prévu une multiplicité de saillies de butée (11) le long de la périphérie de la face annulaire (6 ou 7).

2. Soupape antifuites selon la revendication 1, **caractérisée en ce que** les deux parties de corps (2, 3) dans l'état verrouillé s'appliquent l'une contre l'autre sans jeu de mouvement axial.

3. Soupape antifuites selon la revendication 1, **caractérisée en ce que** les saillies de butée (11) présentent une hauteur située dans une plage de 0,1 mm à 0,3 mm.

4. Soupape antifuites selon la revendication 1, **caractérisée en ce que** les saillies de butée (11) sont en forme de bouton.

5. Soupape antifuites selon au moins une des revendications précédentes, **caractérisée en ce qu'**il est prévu une chambre d'accumulation (13) dans la région du canal de fuite (9).

6. Soupape antifuites selon au moins une des revendications précédentes, **caractérisée en ce que** la région de la partie de corps intérieure (3), qui présente les ouvertures de passage, est parallèle à l'axe longitudinal du corps de soupape.
